(19) Europäisches Patentamt
European Patent Office
Office européen des brevets

(11) EP 1 726 302 A2

(12) # EUROPÄISCHE PATENTANMELDUNG

(43) Veröffentlichungstag:
**29.11.2006 Patentblatt 2006/48**

(51) Int Cl.:
***A61K 9/70*** *(2006.01)* ***A61K 31/4468*** *(2006.01)*

(21) Anmeldenummer: **06017854.8**

(22) Anmeldetag: **03.02.2004**

(84) Benannte Vertragsstaaten:
**AT BE BG CH CY CZ DE DK EE ES FI FR GB GR HU IE IT LI LU MC NL PT RO SE SI SK TR**

(30) Priorität: **07.02.2003 DE 10304989**
**22.01.2004 DE 102004003224**

(62) Dokumentnummer(n) der früheren Anmeldung(en) nach Art. 76 EPÜ:
**04707525.4 / 1 589 955**

(71) Anmelder: **LTS LOHMANN Therapie-Systeme AG**
**56626 Andernach (DE)**

(72) Erfinder: **Müller, Walter, Dr.**
**56626 Andernach (DE)**

(74) Vertreter: **Schmidt, Werner**
**LTS Lohmann**
**Therapie-Systeme AG**
**Patentabteilung**
**Postfach 15 25**
**56605 Andernach (DE)**

Bemerkungen:
Diese Anmeldung ist am 26 - 08 - 2006 als Teilanmeldung zu der unter INID-Kode 62 erwähnten Anmeldung eingereicht worden.

(54) **Für die Wärmeanwendung zur Beschleunigung der Permeation von Wirkstoffen geeignetes transdermales therapeutisches System und seine Verwendung**

(57) Die vorliegende Erfindung betrifft ein Transdermales Therapeutisches System (TTS) enthaltend mindestens einen pharmakologischen Wirkstoff, welches geeignet ist für eine Erwärmung auf eine über der Hauttemperatur liegende Temperatur zur Steigerung der Permeationsrate des Wirkstoffs, umfassend mindestens eine wirkstoffundurchlässige Rückschicht, mindestens eine den Wirkstoff enthaltende Schicht und eine abziehbare Rückschicht, das dadurch gekennzeichnet, dass die wirkstoffhaltige Schicht vorwiegend aus einem Polysiloxan besteht, worin der Wirkstoff teilweise gelöst vorliegt.

EP 1 726 302 A2

## Beschreibung

Transdermale Therapeutische Systeme (TTS) sind mittlerweile eine gut eingeführte Arzneiform geworden, die wegen ihrer therapeutischen Vorteile auch von den Patienten gut akzeptiert wird. Aufgrund der Barrierewirkung der menschlichen Haut ist allerdings die transdermale Verabreichung von Wirkstoffen mit topischer und systemischer Wirkung auf Substanzen beschränkt, die auf Grund ihrer Wirksamkeit bei schon geringen Dosen und ihrer physikalisch-chemischen Eigenschaften auf einer akzeptablen Fläche in pharmakologisch ausreichender Menge durch die Haut permeieren können.

Es gab und gibt deshalb zahlreiche Anstrengungen die Barrierewirkung der Haut zu beeinflussen und dadurch das Spektrum der für die transdermale Therapie geeigneten Wirkstoffe zu erweitern. Ein erster Ansatz dieses Ziel zu erreichen war die Verwendung von speziellen Substanzen, den sogenannten Permeationsenhancern, die ebenfalls in die Haut eindiffundieren und die Struktur des Stratum Comeums, der Hauptdiffusionsbarriere, beeinflussen. Beispielhaft seien genannt Fettalkohole, Fettsäuren und Fettsäureester, Dimethylsulfoxid, Glycerinderivate, polyoxyethylierte Fettalkohole und Fettsäuren.

Neben diesem sogenannten chemischen Enhancement wurden auch physikalische Verfahren wie elektrischer Strom bei der sogenannten lontophorese, Ultraschall bei der sogenannten Sonophorese und Wärme erfolgreich eingesetzt.

Das einfachste physikalische Mittel, um die Durchlässigkeit der Haut für chemische Substanzen zu steigern, ist die Einwirkung von Wärme.

Ein transdermales System, das sich diesen Effekt zu Nutze macht, ist in der US Patentschrift 4,898,592 beschrieben. Das dort beschriebene transdermale System enthält optional eine wärmeleitende Schicht, um die Körperwärme zur Erhöhung der Temperatur an der Applikationsstelle des TTS zu nutzen.

Während dieses System rein passiv arbeitet, ist in der US-Patentschrift 4,230,105 ein System beschrieben, das nach Aktivierung mit Wasser aktiv Wärme entwickelt und dadurch die Permeationsrate für Wirkstoffe steigert.

In der US-Patentschrift 5,919,479 ist ein Prinzip beschrieben, das die Anwendung von kontrollierter Wärme in Kombination mit der Anwendung von Lokalanästhetika offenbart. Die Wärme wird dabei durch die Reaktion von Luftsauerstoff mit auf Aktivkohle befindlichem pyrophorem Eisen in Gegenwart von Wasser erzeugt.

In der US-Patentschrift 6,261,595 wird beschrieben, wie wärmeerzeugende Vorrichtungen mit konventionellen transdermalen Systemen vorteilhaft kombiniert werden können. Es wird darauf hingewiesen, daß es besonders vorteilhaft ist, wenn das transdermale Systeme so beschaffen ist, daß das Wärme erzeugende bzw. -abgebende Element jederzeit angebracht, entfernt bzw. ausgetauscht werden kann, ohne das transdermale System zu schädigen. Die leichte Austausch- bzw. Entfernbarkeit ist speziell wichtig, wenn die Wirkstoffabgabe an die jeweiligen Bedürfnisse des Patienten angepasst werden soll. Dies ist z.B. besonders wünschenswert, wenn es sich bei dem Wirkstoff um ein Schmerzmittel handelt. Besonders bei nebenwirkungsreichen Schmerzmitteln wie Opiaten, Fentanyl und fentanylanalogen Substanzen sollte der Patient einerseits jeweils eine ausreichende Menge zugeführt bekommen, um beschwerdefrei zu sein, andererseits sollte wegen der Nebenwirkungen auch nicht mehr als die der jeweiligen Situation angemessene Menge verabreicht werden. Durch die Anwendung von Wärme kann bei auftretenden Schmerzen die Wirkstoffabgabe des Systems zeitweise erhöht und damit dem gestiegenen Bedarf angepasst werden.

In keiner der obigen Patentschriften wird jedoch auf die besonderen Anforderungen an die Eigenschaften der Matrix, den wirkstoffhaltigen Teil von transdermalen Systemen, bei Anwendung von Wärme zur Erhöhung der Permeationsrate eingegangen.

Aufgabe dieser Erfindung ist es deshalb, Formulierungen für die wirkstoffhaltigen Teile eines Matrixsystems zu entwickeln, die besonders gut geeignet sind, die Wirkstoffabgabe für die ganze oder einen Teil der Applikationszeit durch die Anwendung von Wärme zu erhöhen.

Matrixsysteme bestehen im einfachsten Fall lediglich aus einer wirkstoffundurchlässigen Rückschicht, einer wirkstoffhaltigen und idealerweise selbstklebenden Matrixschicht und einer vor Gebrauch zu entfernenden Schutzschicht. Die wirkstoffhaltige Schicht besteht üblicherweise neben dem Wirkstoff zum größten Teil aus Polymeren auf der Basis von Polyacrylaten, Polyisobutylen, Polyisopren, Blockpolymeren aus Styrol-Polybutylen-Styrol, Polysiloxanen, Polyurethanen oder Hydrogelen. Speziell Matrices auf Basis von Polyisobutylen und Blockpolymeren enthalten zusätzliche Klebrigmacher auf der Basis von Kohlenwasserstoffharzen und/ oder Kolophoniumderivaten. Alle Matrices können zusätzliche Hilfsstoffe enthalten, die die Löslichkeit des Wirkstoffs beeinflussen bzw. Permeationsenhancer, die die Absorptionsrate aus dem System erhöhen.

Bei Matrixsystemen erfolgt die Steuerung der Wirkstoffaufnahme üblicherweise weniger durch das System, sondern zu einem großen Teil durch die Haut selbst. Allerdings gibt es die Möglichkeit, die Matrix hautseitig mit einer Steuermembran und die Membran gegebenenfalls mit einer Kleberschicht zur Verankerung des Systems auf der Haut zu versehen um dadurch eine bessere Systemkontrolle zu erreichen.

Die hautseitige Kleberschicht zur Verankerung auf der Haut kann ebenfalls mit Wirkstoff beladen werden. Dieser Anteil wird dann ohne Membrankontrolle abgegeben um z.B. ein mögliches Hautdepot für den Wirkstoff möglichst

schnell zu sättigen.

Für die in-vivo Wirkstoffabgabe bei einfachen monolithischen Matrixsystemen ist neben den physikalisch-chemischen Eigenschaften des Wirkstoffs und der Anwesenheit und Wirksamkeit von chemischen Permeationsenhancern die thermodynamische Aktivität des Wirkstoffs der entscheidende, die Abgaberate bestimmende Parameter. In einem stabilen System ist die maximale thermodynamische Aktivität erreicht, wenn die Konzentration des Wirkstoffs der Sättigungskonzentration des Wirkstoffs in der Matrixformulierung entspricht. Eine weitere Erhöhung der Wirkstoffkonzentration führt zu keiner weiteren Steigerung der thermodynamischen Aktivität, wenn der die Sättigungslöslichkeit übersteigende Anteil des Wirkstoffs als reiner Wirkstoff, d.h. in Form von Kristallen, bzw. bei niedrig schmelzenden Wirkstoffen als Flüssigphase, in der Matrix vorliegt. Ist der Wirkstoff in einer die Sättigungskonzentration übersteigenden Menge in dem System gelöst, spricht man von übersättigten Systemen. Da der Wirkstoff in solchen Systemen dazu neigt, während der Lagerzeit zu rekristallisieren bzw. eine eigene Phase zu bilden, müssen solche Systeme als meta- bzw. instabil betrachtet werden.

Die oben geschilderten Zusammenhänge zeigen, daß es eine komplexe Wechselwirkung zwischen der aktuellen Wirkstoffkonzentration und den Löseeigenschaften der jeweiligen Matrixformulierung gibt. Diese Wechselwirkungen müssen speziell bei transdermalen Systemen, bei denen die Wirkstoffabgabe während der Applikationszeit oder zumindest bei einem Teil der Applikationszeit durch die Anwendung von Wärme gesteigert wird, berücksichtigt werden.

An ein solches System sind folgende Forderungen zu stellen:

1. Die thermodynamische Aktivität des Wirkstoffs sollte nach der Anwendung von externer Wärme unverändert sein

2. Die thermodynamische Aktivität des Wirkstoffs muss nach der Anwendung von Wärme schnell auf das Ausgangsniveau zurückgehen.

3. Das System muß so ausgelegt sein, dass auch bei Anwendung von Wärme und der damit verbundenen zeitweise erhöhten Abgaberate die Wirkstoffabgabe für die gesamte Applikationszeit auf dem gewünschten Niveau gewährleistet ist und sich das System nicht vorzeitig erschöpft..

Die Aufgabe wird erfindungsgemäß durch ein transdermales therapeutisches System gemäß Anspruch 1 der vorliegenden Anmeldung gelöst.

Gegenstand der Anmeldung ist daher ein für die Verabreichung mindestens eines pharmakologischen Wirkstoffes in einem oberhalb der Hauttemperatur liegenden Temperaturbereich geeignetes transdermales therapeutisches System (TTS), umfassend eine wirkstoffundurchlässige Rückschicht, mindestens eine wirkstoffhaltige Matrixschicht auf der Basis von mindestens einem Polysiloxan, eine abziehbare Schutzschicht, sowie ein internes und/oder externes wärmeabgebendes Element, dadurch gekennzeichnet, das

a) die wirkstoffhaltige Schicht ohne die Berücksichtigung des Wirkstoffes zu mindestens 80 Gew.-% aus Polysiloxan besteht,
b) der Wirkstoff zu höchstens 20 Gew.-% in gelöster Form vorliegt, und
c) der Wirkstoff in dem Polysiloxan eine Sättigungslöslichkeit von höchstens 5 Gew.-% besitzt.

In einer Ausführungsform enthält das erfindungsgemäße TTS, z. B. in Form einer Matrixschicht ein internes wärmeabgebendes Element.
Eine bevorzugte Ausführungsform der vorliegenden Erfindung ist ein TTS, das mit einem externen wärmeabgebenden Element, einem sogenannten Heatpack verbunden ist, welches entfernt bzw. ausgetauscht werden kann, ohne das transdermale System zu schädigen. Dieses Element kann gemäß einer weiteren Ausführungsform auch getrennt vom TTS, aber in einer gemeinsamen Verpackungseinheit vorliegen.

Gegenstand der vorliegenden Erfindung ist ferner die Verwendung des oben definierten TTS zur transdermalen Verabreichung von Wirkstoffen bei Temperaturen höher als die Hauttemperatur, dadurch gekennzeichnet, dass nach Entfernung der Schutzschicht die von der Rückschicht bedeckte Matrixschicht auf die Haut aufgebracht wird und entweder durch ein internes wärmeabgebendens Element oder durch Verbinden des TTS mit einem externen wärmeabgebenden Element (sogenanntes Heatpack) die Matrixschicht für eine bestimmte Zeit auf eine Temperatur, die höher als die Hauttemperatur ist, erwärmt wird. In einer bevorzugten Ausführungsform der Verwendung wird die Matrix auf eine Temperatur bis einschließlich 50°C erwärmt.

Gegenstand der vorliegenden Erfindung ist ferner eine Verpackungseinheit, in welcher das TTS sowie das externe wärmeabgebende Element (Heatpack) getrennt von einander verpackt sind.

Erfindungsgemäße interne oder externe wärmeabgebende Elemente können solche sein, wie sie z. B. in den US-Patentschriften 5,919,479 bzw. 6,261,595 beschrieben worden sind.

Polysiloxane (genauer: Polyorganosiloxane) sind in der Form von Lösungen oder als lösemittelfreie Zweikomponentensysteme erhältlich. Durch die Variation des organischen Teils der Polysiloxane gibt es eine Vielzahl von unterschiedlichen Polysiloxanen, wobei jedoch für transdermale Systeme bisher nur Polydimethylsiloxane eingesetzt werden.

Bei Zweikomponentensystemen hat das Polysiloxan ein relativ geringes Molekulargewicht und ist bei Raumtemperatur flüssig bis zähflüssig. Erst beim Zusammengeben der beiden Komponenten erfolgt eine Quervernetzung die je zu einem gebrauchsfähigen Produkt führt.

Für die Herstellung von transdermalen Systemen werden bevorzugt gelöste Polydimethylsiloxane gemäß folgender Strukturformel eingesetzt.

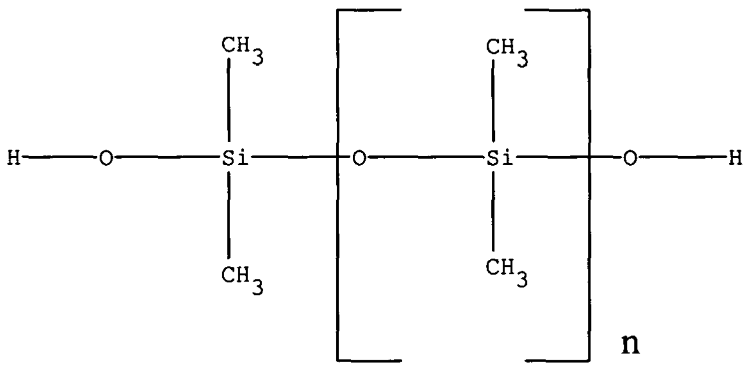

Durch die zusätzliche Verwendung von Monomethylsiloxan als Monomer wird dabei eine gewisse dreidimensionale, die Kohäsion verbessernde, Vernetzung erreicht.

In weiteren erfindungsgemäßen Polysiloxanen können die Methylgruppen ganz oder teilweise durch andere Alkyl- bzw. Phenylreste ersetzt sein.

Das Molekulargewicht und der Grad der Quervernetzung sind wichtige Parameter, die die Kohäsion und die Klebrigkeit des Polymers nach Entfernung des Lösemittels bestimmen.

Auf Grund der freien OH-Gruppen neigen Polysiloxane gemäß obiger Strukturformel in Gegenwart von basischen Substanzen, z. B. Aminen, zu Kondensationsreaktionen, die im Laufe der Zeit die Klebrigkeit reduzieren. In einer besonderen aminresistenten Form dieser Polymere sind deshalb alle freien Si-OH Gruppen in Si-O- C $(CH_3)_3$ Gruppen umgewandelt.

Generell zeichnen sich Polysiloxane durch eine gute Hautverträglichkeit, eine geringes Lösevermögen für die meisten Wirkstoffe und einen extrem hohen Diffusionskoeffizienten für den gelösten Anteil des Wirkstoffs aus. Im Sinne dieser Erfindung ist speziell die geringe Lösekapazität und der hohe Diffusionskoeffizient von besonderer Bedeutung.

Wegen der geringen Lösekapazität ist selbst bei einer geringen Wirkstoffbeladung der größte Teil des Wirkstoffs ungelöst. Durch geeignete Wahl des Verhältnisses von Wirkstoff zu Polysiloxan kann in jedem Fall dafür gesorgt werden, daß bei einer Löslichkeit des Wirkstoffs von nicht mehr als 5 Gew. % in der Matrix mindestens 80 % des Wirkstoffs ungelöst vorliegen. Da deshalb nur ein kleiner Teil der während der Applikationszeit abzugebenden Menge an Wirkstoff in gelöster Form in der Matrix vorliegt, ist das Nachlösen des Wirkstoffs und seine schnelle Diffusion zur Abgabefläche wichtig, um die Konzentration des gelösten Wirkstoffs in der Matrix in der Nähe der Sättigungskonzentration zu halten und damit eine konstant hohe thermodynamische Aktivität des Wirkstoffs über den Applikationszeitraum zu gewährleisten. Polysiloxane erfüllen auch diese Bedingung in idealer Weise auf Grund ihrer hohen Diffusibilität.

Bei der externen Anwendung von Wärme wird das TTS und damit die Matrix auf Temperaturen bis zu 50 °C erwärmt. Es ist dabei ausnahmslos davon auszugehen, daß aus der Temperaturerhöhung eine Erhöhung der Sättigungslöslichkeit des Wirkstoffs und eine Erhöhung der Konzentration des gelösten Wirkstoffs resultiert. Da sich dadurch die thermodynamische Aktivität des Wirkstoffs in der Matrix und der Diffusionskoeffizient des Wirkstoffs im Stratum Corneum erhöht, steigt die Abgabegeschwindigkeit des Wirkstoffs aus dem TTS an den Organismus.

Da bei Rückkehr zu Normalbedingungen (d. h.: Hauttemperatur) die Menge an gelöstem Wirkstoff zunächst unverändert bleibt, ist die Matrix wegen der nun wieder erniedrigten Sättigungslöslichkeit übersättigt. Da dieser Effekt zu einer nun bei Hauttemperatur ebenfalls erhöhten thermodynamischen Aktivität und damit einem erhöhten Wirkstoffflux durch die Haut führt, ist es wichtig, dass die Übersättigung schnell abgebaut wird. Polysiloxane als Grundpolymer für Matrices erfüllen diese Bedingung wegen ihrer schlechten Löslichkeit für Wirkstoffen in idealer Weise, da auf Grund der niedrigen Sättigungslöslichkeit von Wirkstoffen in solchen Matrices die Rückkehr der Konzentration des angelösten Wirkstoffes zur Sättigungskonzentration durch die Wirkstoffabgabe an den Organismus innerhalb kurzer Zeit erreicht wird. Eine Rückkehr zur Sättigungslöslichkeit durch eine Rekristallisation des über der Sättigungslöslichkeit gelösten

Anteils Wirkstoffs ist nur theoretisch möglich, da in Polymeren Wirkstoffe bzw. andere gelöste Substanzen nur schwer bzw. sehr langsam auskristallisieren.

Der oben geschilderte Sachverhalt kann durch eine Modellrechnung an 2 typischen TTS verdeutlicht werden. Dabei wird ein TTS, das den ungünstigsten Fall eines TTS auf Basis eines Silikonklebers im Sinne dieser Erfindung darstellt mit einem durchaus typischen TTS auf Basis von Polyacrylatklebern verglichen.

**Annahmen:**

| | |
|---|---|
| TTS Fläche: | 10 $cm^2$ |
| Wirkstoffbeladung: | 20 mg/ TTS |
| Sättigungslöslichkeit in Silikonsystem: | 5 % (g/g) |
| Beschichtungsgewicht Silikonsystem: | 80 g/ $m^2$ |
| Sättigungslöslichkeit in Polyacrylatsystem: | 20 % |
| Beschichtungsgewicht Polyacrylatsystem: | 50 g/ $m^2$ |
| Wirkstoffabgabe: | 50 μg/ h |
| Temperaturerhöhung: | 32 °C → 40 °C |
| Erhöhung der Sättigungslöslichkeit bei 40 °C: | 10 % |

| | **Silikon-TTS** | **Polyacrylat-TTS** |
|---|---|---|
| Gewicht der Matrix | 16 mg | 10 mg |
| Wirkstoffbeladung | 4 mg | 4 mg |
| davon gelöst bei 32 °C | 0,8 mg [1)] | 2 mg [2)] |
| davon gelöst bei 40 °C | 0,88 mg | 2,2 mg |
| Zeit bis zur Rückkehr zur Sättigungslöslichkeit bei 32 °C [3)] | 1,6 Stunden | 4,0 Stunden |

1) Sättigungslöslichkeit 5 Gew. %; entspricht 20 % der Gesamtwirkstoffmenge
2) Sättigungslöslichkeit 20 Gew. %, entspricht 50 % der Gesamtwirkstoffmenge
3) Berechnung:

$$\frac{(\text{mg Wirkstoff gelöst bei 40 °C} - \text{mg Wirkstoff gelöst bei 32 °C}) * 1000}{\text{Abgaberate } [\,\mu g/\,h\,]}$$

Etwas vereinfacht wurde dafür angenommen, daß die Wirkstoffabgabe unmittelbar nach Ende der Wärmeanwendung wieder 50 μg/ h Stunde beträgt.

Qualitative sind die Unterschiede im Verhalten von beiden TTS in folgender Zeichnung verdeutlicht.

Figur 1: Verhalten von Silikon- und Polyacrylat-TTS nach Anwendung von Wärme zur Steigerung der Permeationsrate

Bei einem TTS, das schon anfänglich den Wirkstoff unterhalb der Sättigungskonzentration enthält, kann sich kein Wirkstoff nachlösen, wenn infolge der Temperaturerhöhung die Sättigungslöslichkeit steigt und die Differenz zwischen tatsächlicher Konzentration und Sättigungskonzentration größer wird. Dadurch fällt die Erhöhung der Permeationsrate im Vergleich zu gesättigten Systemen schwächer aus, wobei die Erhöhung des Diffusionskoeffizienten im Stratum Corneum den wesentlichen Beitrag leistet.

Wenn bei solchen Systemen die Permeationsrate durch Erwärmung erhöht wird, wird durch die verstärkte Wirkstoffabgabe während der Wärmeanwendung die Wirkstoffkonzentration schneller erniedrigt. Bei Rückkehr zu Normalbedingungen bedeutet dies, daß die Wirkstoffabgabe in Abhängigkeit von der zusätzlich abgegebenen Wirkstoffmenge erniedrigt ist. Damit sind solche untersättigten Systeme weitgehend unbrauchbar in Verbindung mit Wärmeanwendungen zur Steigerung der Permeationsraten, wenn eine konstante Wirkstoffabgabe über den Applikationszeitraum zwischen den Wärmeanwendungsphasen gefordert ist. Lediglich Systeme mit einer hohen Wirkstoffbeladung und einer geringen Wirkstoffabgabe sind unter dieser Bedingung geeignet, da sich unter diesen Bedingungen die Wirkstoffkonzentration und damit die Abgaberate während der Applikationszeit bzw. durch die gesteigerte Wirkstoffabgabe während einer Hitzeanwendung relativ wenig ändert.

Erkauft wird sich dies allerdings dann mit einer geringeren Wirkstoffausnutzung und bei teuren Wirkstoffen sind solche Systeme unwirtschaftlich.

TTS im Sinne dieser Erfindung können hergestellt werden, indem der Wirkstoff in der Lösung des Polysiloxans suspendiert wird, alle anderen Hilfsstoffe zugegeben werden und die Masse durch Rühren homogenisiert wird. Nach dem Beschichten auf eine geeignete Folie wird das Lösemittel des Klebers abgedampft und der getrocknete Film mit der Rückschicht des TTS kaschiert. Aus diesem Laminat werden dann die fertigen TTS gestanzt. Die gesamte Prozedur wird an Beispiel 1 mit Fentanyl als Base exemplarisch dargestellt. In Beispiel 2 wird die Herstellung eines TTS mit einer zusätzliche Steuermembran beschrieben.

Als Wirkstoffe, die für das erfindungsgemäße transdermale therapeutische System infrage kommen, seien die folgenden genannt:

Analgetika, wie z. B. Fentanyl oder eine fentanylanaloge Substanz, Butorphanol, Oxicodon, Ketorolac, Buprenorphin, Morphin, Tetracain, Lidocain, Bupivacain, Prilocain und Benzocain;
Antiparkinson-Medikamente wie z. B.Biperiden, Selegillin, Pramipexol Broncholytika wie z. B. Salbutamol, Tulobuterol Antiepileptika wie z. B. Clonazepam, Tiagabin

**Beispiel 1: TTS mit Fentanyl als Wirkstoff ohne Steuermembran**

3 g Fentanyl Base werden zu 138 g einer 70 prozentigen Lösung eines Silikonklebers in n-Heptan (BIO-PSA 4301, Dow Corning) gegeben und durch Rühren in dieser Lösung suspendiert. Die Suspension wird dann auf eine geeignete wiederentfernbare Schutzfolie (Scotchpak 1022, 3M) in einer Dicke beschichtet, daß nach dem Entfernen des Lösemittels ein Beschichtungsgewicht von 100 g/m$^2$ resultiert. Der getrocknete Film wird nun mit einer 23 $\mu$m dicken Polyesterfolie, der Rückschicht des TTS, kaschiert und aus diesem Laminat die TTS gestanzt. Die einzelnen TTS werden in einem Beutel aus einem heißsiegelbaren Packstofflaminat verpackt.

**Beispiel 2: TTS mit Fentanyl als Wirkstoff mit Steuermembran**

**a. Herstellung der Reservoirschicht**

3 g Fentanyl Base werden zu 138 g einer 70 prozentigen Lösung eines Silikonklebers in n-Heptan (BIO-PSA 4301, Dow Corning) gegeben und durch Rühren in dieser Lösung suspendiert. Die Suspension wird dann auf eine geeignete wiederentfernbare Schutzfolie (Scotchpak 1022, 3M) in einer Dicke beschichtet, daß nach dem Entfernen des Lösemittels ein Beschichtungsgewicht von 100 g/ m$^2$ resultiert. Der getrocknete Film wird nun mit einer 23 $\mu$m dicken Polyesterfolie, der Rückschicht des TTS, kaschiert.

**b. Herstellung der Hautkleberschicht**

Die Lösung eines Silikonklebers mit einem Feststoffgehalt von 70 % in n-Heptan (BIO-PSA 4301, Dow Corning) wird auf eine geeignete wiederentfernbare Schutzfolie (Scotchpak 1022, 3M) in einer Dicke beschichtet, daß nach dem Entfernen des Lösemittels ein Beschichtungsgewicht von 30 g/ m$^2$ resultiert. Auf den getrockneten Film wird nun die Steuermembran (50 $\mu$m dicke EVA-Film mit einem Vinylacetagehalt von 19%) aufkaschiert.

**c. Herstellung des Gesamtlaminats**

Von der unter a hergestellten Reservoirschicht wird die wiederentfernbare Schutzfolie abgezogen und auf die Membran der unter b hergestellten Hautkleberschicht aufkaschiert. Aus dem Gesamtlaminat werden die Einzel-TTS gestanzt und in einem Beutel aus einem heißsiegelbaren Packstofflaminat verpackt.

**Patentansprüche**

1. Transdermales Therapeutisches System (TTS) für die Verabreichung mindestens eines Wirkstoffes, umfassend

eine wirkstoffundurchlässige Rückschicht, mindestens eine wirkstoffhaltige Matrixschicht auf der Basis von mindestens einem Polysiloxan, und eine abziehbare Schutzschicht, **dadurch gekennzeichnet, dass**

a) die wirkstoffhaltige Matrixschicht zu mindestens 80 Gew.-% aus mindestens einem Polysiloxan besteht,
b) der Wirkstoff in der wirkstoffhaltigen Matrixschicht zu höchstens 20 % in gelöster Form vorliegt, und
c) der Wirkstoff in dem Polysiloxan eine Sättigungslöslichkeit von höchstens 5 Gew.-% besitzt.

**2.** TTS nach Anspruch 1, **dadurch gekennzeichnet, dass** das Polysiloxan ein Polydimethylsiloxan ist.

**3.** TTS nach Anspruch 1 oder 2, **dadurch gekennzeichnet, dass** das Polysiloxan ein gelöstes Polydimethylsiloxan gemäß der Strukturformel

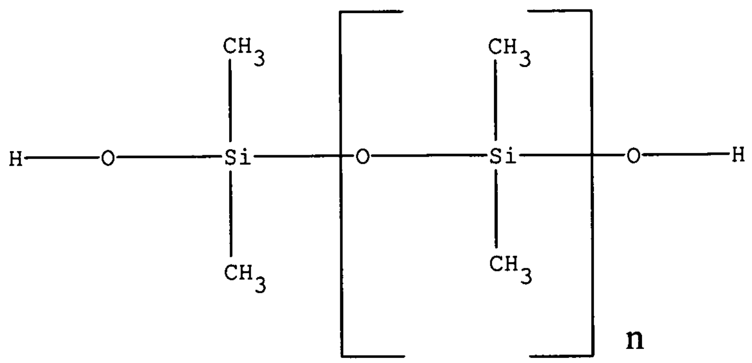

ist.

**4.** TTS nach einem oder mehreren der vorangehenden Ansprüche, **dadurch gekennzeichnet, dass** das Polysiloxan vernetzt ist.

**5.** TTS nach einem oder mehreren der vorangehenden Ansprüche, **dadurch gekennzeichnet, dass** bei dem Polysiloxan die Methylgruppen ganz oder teilweise durch andere Alkyl- oder Phenylreste ersetzt sind.

**6.** TTS nach einem oder mehreren der vorangehenden Ansprüche, **dadurch gekennzeichnet, dass** das Polysiloxan aminresistent ist.

**7.** TTS nach einem oder mehreren der vorangehenden Ansprüche, **dadurch gekennzeichnet, dass** das Polysiloxan selbstklebend ist.

**8.** TTS nach einem oder mehreren der vorangehenden Ansprüche, **dadurch gekennzeichnet, dass** der Wirkstoff in der wirkstoffhaltigen Matrixschicht die maximale thermodynamische Aktivität erreicht.

**9.** TTS nach einem oder mehreren der vorangehenden Ansprüche, **dadurch gekennzeichnet, dass** mindestens eine wirkstoffhaltige Matrixschicht hautseitig mit einer Steuermembran und diese gegebenenfalls mit einer Kleberschicht zur Verankerung auf der Haut versehen ist.

**10.** TTS nach Anspruch 9, **dadurch gekennzeichnet, dass** die Kleberschicht zur Verankerung auf der Haut mit Wirkstoff beladen ist.

**11.** TTS nach einem oder mehreren der vorangehenden Ansprüche, **dadurch gekennzeichnet, dass** die wirkstoffhaltige Matrixschicht einen die Barriereeigenschaften der Haut reduzierenden Hilfsstoff enthalten.

**12.** TTS nach Anspruch 11, **dadurch gekennzeichnet, dass** der die Barriereeigenschaften der Haut reduzierende Hilfsstoff aus der Gruppe bestehend aus Fettalkoholen, Fettsäuren, Fettsäureester, Dimethylsulfoxid, Glycerinderivate, polyoxyethylierte Fettalkohole und polyoxyethylierte Fettsäuren ausgewählt ist.

**13.** TTS nach Anspruch 12, **dadurch gekennzeichnet, dass** der die Barriereeigenschaften der Haut reduzierende

Hilfsstoff Ölsäure ist.

**14.** TTS nach einem oder mehreren der vorangehenden Ansprüche, **dadurch gekennzeichnet, dass** der Wirkstoff Fentanyl ist.

**15.** TTS nach einem oder mehreren der vorangehenden Ansprüche, **dadurch gekennzeichnet, dass** der Wirkstoff eine fentanyl-analoge Substanz, Butorphanol, Oxycodon, Ketorolac, Buprenorphin, Morphin, Tetracain, Lidocain, Bupivacain, Prilocain oder Benzocain ist.

**16.** TTS nach einem oder mehreren der vorangehenden Ansprüche, **dadurch gekennzeichnet**, das der Wirkstoff ein Antiparkinsonmittel, ein Impfserum, ein Broncholytikum, ein Antiepileptikum oder ein Suchtentwöhnungsmittel ist.

**17.** TTS nach Anspruch 14, **dadurch gekennzeichnet, dass** es aus (a) einer 23 μm dicken Polyesterfolie als wirkstoffundurchlässiger Rückschicht, (b) einer wirkstoffhaltigen Matrixschicht, die durch Zugabe von 3 g Fentanyl Base zu 138 g einer 70%-igen Lösung eines Silikonklebers in n-Heptan, Rühren, Suspendieren und Beschichten in einer Dicke, dass nach Entfernen des Lösemittels ein Beschichtungsgewicht von 100 $g/m^2$ resultiert, hergestellt wird, und (c) einer abziehbaren Schutzschicht besteht.

**18.** TTS nach Anspruch 17, **dadurch gekennzeichnet, dass** es zusätzlich eine Steuermembran aus einem 50 μm dicken EVA-Film mit einem Vinylacetatgehalt von 19% und eine Kleberschicht zur Verankerung auf der Haut besitzt, welche durch Beschichten einer 70%-igen Lösung eines Silikonklebers in n-Heptan in einer Dicke, dass nach Entfernen des Lösemittels ein Beschichtungsgewicht von 30 $g/m^2$ resultiert, hergestellt wird.

**19.** Verfahren zur Herstellung eines TTS durch die Schritte:

a) Suspendieren des Wirkstoffs in der Lösung des Polysiloxans,
b) Homogenisieren der Suspension durch Rühren,
c) Beschichten auf eine Folie
d) Abdampfen des Lösungsmittels
e) Kaschieren des getrockneten Films mit der Rückschicht
f) Ausstanzen

**20.** Verfahren nach Anspruch 19, wobei der Wirkstoff Fentanyl ist.

**21.** Verwendung eines TTS nach einem der Ansprüche 1 bis 18 zur transdermalen Verabreichung von Wirkstoffen.

**22.** Verwendung nach Anspruch 21 bei Temperaturen, die höher als die Hauttemperatur sind, wobei die wirkstoffhaltige Matrixschicht durch ein internes wärmeabgebendes Element für eine bestimmte Zeit auf eine Temperatur erwärmt wird, die höher als die Hauttemperatur ist.

**23.** Verwendung nach Anspruch 21 bei Temperaturen, die höher als die Hauttemperatur sind, wobei die wirkstoffhaltige Matrixschicht durch ein externes wärmeabgebendes Element für eine bestimmte Zeit auf eine Temperatur erwärmt wird, die höher als die Hauttemperatur ist.

**24.** Verwendung nach Anspruch 22 oder 23, wobei die Matrixschicht auf eine Temperatur bis einschließlich 50 °C erwärmt wird.

**25.** Verwendung nach einem oder mehreren der Ansprüche 21 bis 24, wobei der Wirkstoff Fentanyl ist.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4898592 A
- US 4230105 A
- US 5919479 A
- US 6261595 B